# EUROPEAN PATENT APPLICATION

(11) **EP 1 219 636 A2**
(43) Date of publication of application: **03.07.2002**
(21) Application number: 01420032.3
(22) Date of filing: 13.02.2001
(51) Int. Cl.: C07K 14/505, C12N 15/11, C07H 5/06

(54) **Modified muteins of erythropoietin (EPO) derived from in vitro or in vivo expression system of microorganisms**

(30) Priority: 27.12.2000 KR 2000082531
(71) Applicant: Dreambiogen Co., Ltd., Kangnam-gu, Seoul 135-716 (KR)
(72) Inventor: Choi, Cha Yong, Gwanak-Gu, Seoul 151-755 (KR); Kang, Sang Hyeon, Gwanak-Gu, Seoul 151-845 (KR); Kang, Taek Jin, Seoul 151-868 (KR); Woo, Ji Hyoung, Incheon 403-807 (KR)
(74) Representative: Moinas, Michel

(57) **Abstract**

The present invention discloses modified muteins of erythropoietin (EPO) with increased biological activity derived from *in vitro* or *in vivo* expression system of microorganism exhibited a prolonged circulating half-life compared to unmodified muteins of EPO produced from microorganisms, and novel methods of preparing the selectively modified muteins of EPO.

## Description

### FIELD OF INVENTION

The present invention relates to modified muteins of EPO derived from *in vitro* or *in vivo* expression system of microorganism with a prolonged plasma half-life in the circulation, and methods of production of these selectively modified muteins of EPO. The modification of muteins of EPO derived from microorganism with modifiers results in the modified muteins of EPO with increased biological activity relative to unmodified muteins and polypeptide of EPO produced in microorganism.

### BACKGROUND OF THE INVENTION

Erythropoietin (EPO) is a therapeutic glycoprotein currently used to treat anemia associated with several causes including chronic renal failure. EPO is a prime regulator of red blood cell production in mammals and birds. Specifically, this glycoprotein hormone promotes the rapid growth of red blood cell progenitors in marrow, spleen, and fetal liver, and subsequently is required for their terminal differentiation to circulating erythrocytes. Current therapeutic EPO is produced by animal cell culture. The polypeptide of EPO produced by microorganism such as *Escherichia coli* (*E. coli*) has not been used for therapeutic purposes because of the lack of glycans. Therefore in addition to the method of producing muteins of EPO from a microorganism, a modification process is required that involves a combination of *in vitro* protein synthesis and selective modification such as PEGylation or *in vivo* expression and selective modification such as PEGylation. The mutein of EPO is defined herein as a mutant protein with primary structure deviating from that of natural EPO.

Regardless of expression method (i.e. *in vivo* or *in vitro*), administration of the polypeptide of EPO derived from microorganism results in poor biological activity because of the deficiency of oligosaccharide moiety. Increased clearance of the polypeptide of EPO diminishes its therapeutic effects. The polypeptide of EPO is defined herein as a sole peptide part of protein without any glycans. These problems can be reduced by using modified muteins of EPO. Modification of naturally occurring polypeptides which have therapeutic value is often attempted in an effort to increase their biological activity. Increased biological activity is defined herein as a prolonged plasma half-life. Several methods have been employed to increase the biological activity of therapeutic proteins. These methods often focus on increasing the size of the therapeutic agents. For example, the size of a protein can be increased through chemical conjugation with macromolecules or a reagent such as poly(ethylene glycol) (PEG). This procedure, also known as "PEGylation", has been reported with several protein agents (US patent 4,179,337), first as a means to reduce antigenicity, but also as a way to increased biological activity.

Although many techniques have been developed for producing PEG-protein conjugates, the most frequently used modification methods are based on the use of chemical-derivatization methods to achieve changes in the properties of therapeutic macromolecules that cannot be assigned to changes in primary sequences. The primary objective in most chemical-derivatization approaches is to improve bioavailability (particularly with regard to prolonging plasma-residence time) in order to create therapeutic agents with high potency. Conventional modifications including a chemical PEGylation are limited by the nonspecificity of modification because this approach modifies all accessible functional side chain of amino acid having similar chemical reactivity. Of the 20 amino acids normally present in proteins, 13 possess side-chain functional groups capable, in principle, of reaction with chemical reagents. Including the disulphide bond of cystine, and the secondary amide linkage of the polypeptide backbone, there are 14 groupings that are distinguishable on the basis of chemical reactivity in simple systems: thio- (Cys or, rarely, seleno-Cys), methylthio- (Met), disulphide, primary and secondary hydroxyl (Ser, Thr), phenolic hydroxyl (Tyr), indole (Trp), primary amino (Lys, N-terminus), carboxyl (Asp, Glu, C-terminus), guanidine (Arg), primary (Asn, Gln), secondary and tertiary (Pro) amides and imidazole (His). Although a wide variety of chemical agents exhibit some degree of specificity for one or more of these classes of functional group, most reagents modify all accessible groups of a given class.

To improve the selectivity, it is necessary to introduce unnatural amino acids that contain functional side chain specifically reactive to the modifier into the polypeptide. This is made possible by the use of a cell-free protein synthesis technique.

A cell-free protein synthesis has been used as an experimental tool for the investigation of gene expression *in vitro* especially for the proteins that cannot be synthesized *in vivo* because of their toxicity to host cells. The cell-free protein synthesis has been recently re-evaluated as an alternative to the production of commercially important recombinant proteins, which is mainly due to the recent development of a novel reactor system and the extensive optimization of reactor operation conditions (Kim, D.M. et al., Eur. J. Biochem. 239:881-886 (1996); Kigawa, T. et al., FEBS Lett. 442:15-19 (1999)). In addition, various synthetic amino acids besides the 20 natural amino acids can be effectively introduced by this method into protein structures for specially designed purposes (Noren, C.J. et al., Science 244:182-188 (1989)).

A different approach to cell-free protein synthesis is an *in vivo* method with site-directed mutagenesis. Genes encoding polypeptides without free sulfhydryl groups can be mutated to convert a codon corresponding to certain amino acid at the site to be modified into a cysteine coding codon. As a result, cysteine introduced mutein at the site to be modified will contain free sulfhydryl group. This sulfhydryl group can be used as a specific site for modification (US patent 5,206,344).

Modification of therapeutic proteins with synthetic polymers offers two potential advantages. By far the most important is the increase in residence time in plasma to increase bioavailability in blood and perfused tissue. Second, polymers can mask antigenic determinants in nonhuman proteins and also alter the immunogenicity of vaccines. In both cases, polymers are favoured because they can provide a significant increase in effective molecular weight and reduction of interactions with other macromolecules for comparatively little modification of the protein surface. These modifications create new protein surfaces at which interactions with receptors (such as those involved in plasma clearance or immune system recognition) are often changed differentially. Many therapeutic proteins were modified by synthetic polymers. The popularity of poly(ethylene glycol)s (PEGs) is noteworthy; particularly in the range of molecular weights that are commercially available, the hydrophilic nature of the oxyethylene backbone (each unit of which binds 2-3 molecules of water) and the ease of synthesis of monofunctional derivatives from methoxypoly(ethylene glycol).

Several types of chemical linking have been explored. Some polymers (e.g. ethylene/maleic anhydride copolymers) are intrinsically reactive, but most require specific activation by (initially) polyfunctional reagents. Partial substitution of halogen in tri- or di-halotriazines was a popular early method of activating both amino- and hydroxyl-containing polymers, but it has now been largely superseded because of concerns over side reactions and triazine reactivity. Carbonyl diimidazole is a very effective reagent for activating the hydroxyl group in PEGs to an acylimidazole with good reactivity towards protein amino groups. Certain linking strategies give rise to reversible polymer-protein conjugates (e.g. the use of active esters of succinyl-PEG) where the polymer may be removed by hydrolysis. Methods based on disulphide interchange are less widely used for linking synthetic polymers than for preparing protein-protein conjugates. A survey of PEG-modified therapeutic proteins shows that increasing the molecular weight of the protein above ~60kDa usually has a significant effect on plasma clearance, although the magnitude of the effect depends very much on the mechanism of clearance of the unmodified protein and is more predictable when dominated by glomerular filtration than when receptor-mediated processes operate.

### SUMMARY OF THE INVENTION

The present invention relates to modified muteins of EPO produced from microorganism with a prolonged plasma half-life in the circulation, and methods of production of these selectively modified muteins of EPO. Increased biological activity is defined herein as a prolonged plasma half-life (i.e., a longer circulating half-life relative to the unmodified muteins of EPO produced by microorganism). The present invention further relates to methods of producing the modified muteins of EPO with prolonged plasma half-life described herein, and to the methods of their use. The modification of muteins of EPO derived from microorganism with modifiers resulted in the modified muteins of EPO with increased biological activity relative to unmodified muteins and polypeptide of EPO produced in microorganism.

The modified muteins of EPO of the present invention comprise muteins of EPO produced by cell-free protein synthesis or *in vivo* expression of microorganism that has been modified with modifiers such as PEGs, monosaccharides, disaccharides, oligosaccharides, and polysaccharides chemically, enzymatically, or chemo-enzymatically. Chemo-enzymatic modification or chemo-enzymatic reaction is defined herein as the combination of chemical modification/reaction and enzymatic modification/reaction. Modifiers are defined herein as reagents with the reactive groups that are capable of reacting with the functional group of certain introduced amino acid in muteins of EPO produced by cell-free protein synthesis or *in vivo* expression of microorganism. In certain cases, the modifier may be already attached to the unnatural amino acid

Unmodified muteins of EPO are produced by cell-free protein synthesis derived from microorganism, e.g., *E. coli* or cultivation of recombinant microorganism, e.g., *E. coli* strain harboring the EPO cDNA plasmid that possesses the exchanged codon in glycosylation site by site-directed mutagenesis. Unmodified muteins of EPO are selectively modified by routinely used PEGylation.

The mutein of EPO produced by cell-free protein synthesis contains unnatural amino acid with a functional side chain (protected or not protected) specifically reactive to the modifier. This is only made possible by the use of a cell-free protein synthesis technique. The codon corresponding to amino acid residue at the site to be modified is converted to an amber stop codon by site-directed mutagenesis. The new template containing the amber stop codon is expressed by cell-free protein synthesis. Other stop codons and frameshifts by rare codons (Hohsaka, T. et. al., J. Am. Chem. Soc. 118:9778-9779 (1996)) can be used instead of amber stop codon with the same manner as described herein. During cell-free protein synthesis, a suppressor tRNA corresponding to the amber stop codon is included to encode the amber stop codon to certain unnatural amino acid. An unnatural amino acid that is specifically reactive to the modifier is used to site-specific modification. The expressed mutein of EPO is purified and then modified by chemical, enzymatic, or chemo-enzymatic modification.

The mutein of EPO produced by *in vivo* expression of microorganism is modified by the following technique. Muteins of EPO in which one of the amino acids of the mature native sequence of EPO is replaced by a cysteine residue are prepared and conjugated through the replaced cysteine residue to the selected modifiers. These muteins are made via host expression of mutant genes encoding the muteins that have been changed from the genes for the parent proteins by site-directed mutagenesis.

Thus, as results of the work presented herein, muteins of EPO have now been modified to produce the modified muteins of EPO which exhibit increased biological potency relative to unmodified muteins and polypeptide of EPO produced by microorganism.

### BRIEF DESCRIPTION OF DRAWINGS

Figure 1 shows a schematic process of preparing the mutein of EPO with unnatural amino acid site specifically incorporated according to cell-free protein synthesis;
Figures 2a and 2b show the processes of preparing suppressor tRNA by bonding pdCpA-amino acid complex to tRNA(-CA);
Figure 3 shows the expression vector pK7 which codes human EPO cDNA;
Figure 4 shows a schematic process of synthesizing tRNA(-CA) from pSup-ala plasmid; and
Figure 5 shows a process of preparing PEGlayted mutein of EPO by introducing cysteine at the site of 38^{th} using sited-directed mtagenesis and adding PEG thereto.

### DETAILED DESCRIPTION OF THE INVENTION

The present invention relates to modified muteins of EPO with increased biological activity and methods for producing these modified muteins of EPO. Muteins of EPO suitable for modification by the methods described herein are prepared by cell-free protein synthesis or *in vivo* expression, which contain a reactive functional group in the amino acid to be modified. If the modified muteins of EPO with increased biological activity are used as injectable therapeutic agents, it is possible to reduce the frequency of administration.

In one embodiment of the present invention, the muteins of EPO produced by cell-free protein synthesis or microorganism culture are chemically modified by the covalent attachment of a (or more than two) PEG molecule(s) to each molecule of the muteins of EPO. The PEG is attached to a free sulfhydryl group in the muteins of EPO. Since it is noted from the structure of the polypeptide of wild type EPO that the polypeptide does not contain any reactive (free) sulfhydryl group, free sulfhydryl group is introduced at a desired position (or positions) of EPO.

Specifically, two different methods are used to introduce free sulfhydryl group to EPO. The methods are suppression of stop codon by suppressor tRNA charged with unnatural amino acid containing protected functional group, and introduction of cysteine to a desired position using site-directed mutagenesis.

The mutein of EPO produced by cell-free protein synthesis is introduced with unnatural amino acid containing a functional side chain specifically reactive to the modifier. This is made possible by the use of a cell-free protein synthesis technique. The codon corresponding to amino acid residue at the site to be modified is converted to an amber stop codon by site-directed mutagenesis. The new template containing the amber stop codon is expressed by cell-free protein synthesis. During cell-free protein synthesis, a suppressor tRNA corresponding to the amber stop codon is included to decode the amber stop codon to certain unnatural amino acid. An unnatural amino acid that is specifically reactive to the modifier is attached to the suppressor tRNA. The expressed mutein of EPO is purified and then modified by chemical modification such as PEGylation. Examples of unnatural amino acids and analogues that have been successfully incorporated into proteins are reported by Cornish, V.W. et al. (Angew. Chem. Int. Ed. Engl. 34: 621-633 (1995)). The unnatural amino acids may either contain a protected functional group that requires deprotection before reacting with the modifier, or contain a monosaccharide that can be modified with a synthetic polymers via a chemical reaction or with monosaccharides, disaccharides, oligosaccharides, and polysaccharides via a chemical reaction, enzymatic, or chemo-enzymatic reaction.

A different approach to the modification of the mutein of EPO is an *in vivo* method with site-directed mutagenesis. Genes encoding polypeptides without free sulfhydryl groups can be mutated to convert a codon corresponding to certain amino acid at the site to be modified into a cysteine coding codon. As a result, cysteine-introduced polypeptide will contain free sulfhydryl group at the site to be modified. This sulfhydryl group can be used as a specific site for modification. After the preparation of the muteins of EPO, PEGylation is carried out. Muteins of EPO in which one of the amino acids of the mature native sequence of EPO is replaced by a cysteine residue are prepared and conjugated through the replaced cysteine residue to the selected modifiers. These muteins are made via host expression of mutant genes encoding the muteins that have been changed from the genes of the parent proteins by site-directed mutagenesis. The conjugation of the selected modifiers to the mutein of EPO may be made by the same manner as in the cell-free protein system, i.e., the chemical reaction, enzymatic, or chemo-enzymatic reaction.

The method of preparing PEGylated mutein of EPO via combination of cell-free protein synthesis and PEGylation is described in detail in Example 1. And, the method of preparing PEGylated mutein of EPO via combination of *in vivo* expression and PEGylation is described in detail in Example 2.

The present invention will now be more detailedly illustrated by the following examples. However, it will be understood that the present invention is not limited to these specific examples, but is susceptible to various modifications that will be recognized by the skilled person in the present invention.

### Example 1: Production of PEGylated mutein of erythropoietin by cell-free protein synthesis

The lack of cognate tRNA to amber stop codon results in early termination at the amber codon in the mRNA of amber codon containing DNA. However, when amber codon containing DNA was transcribed and then translated in a cell-free protein synthesis system supplemented with cognate suppressor tRNA, full-length polypeptide or protein could be synthesized (Noren, CJ. et al., Science 244:182-188 (1989)).

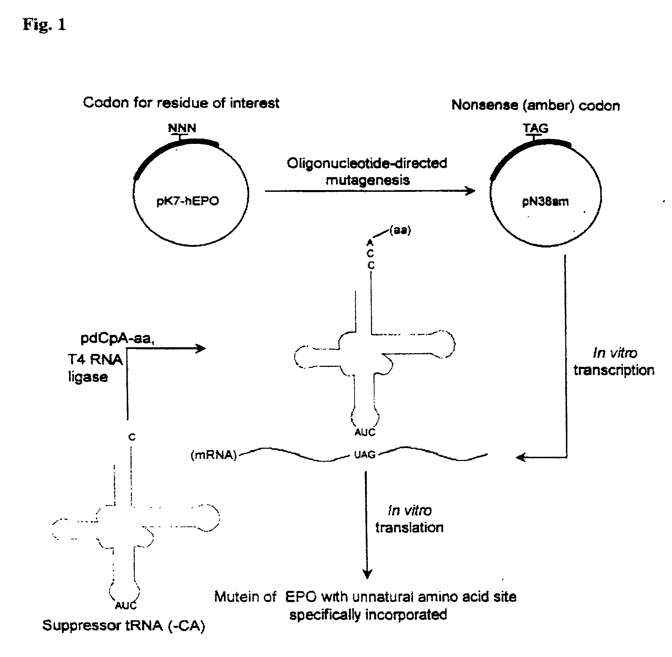

Figure 1 shows the schematic process of preparing the mutein of EPO with unnatural amino acid site specifically incorporated according to cell-free protein synthesis.

The codon of residue of interest in wild type EPO (asparagine) was mutated by site-directed mutagenesis to amber stop codon. This mutein-coding plasmid was translated in a cell-free protein synthesis system supplemented with amber suppressor tRNA charged with unnatural amino acid containing protected functional group. T7 RNA polymerase mediated transcription of this plasmid followed by translation and suppression by suppressor tRNA resulted in the mutein of full-length EPO with unnatural amino acid at desired position. Immuno-purified mutein of EPO was subsequently deprotected to expose free sulfhydryl group at the desired position of EPO and PEGylated as described as follow. Specifically, PEG-modified mutein of EPO at 38^{th} residue was produced as briefly described below.

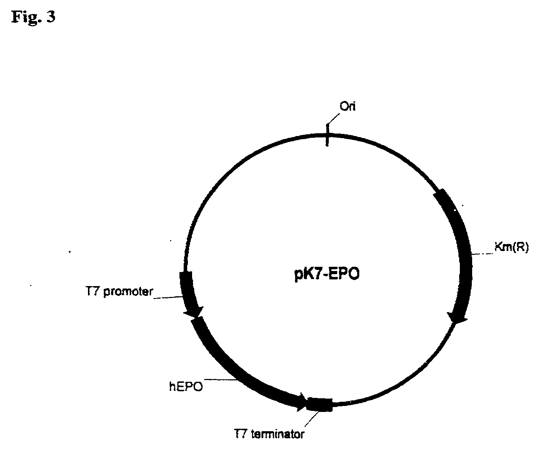

cDNA of wild type EPO was cloned into a prokaryotic expression vector pK7(Kim. D. M. et al., Eur. J. Biochem. 239:881-886 (1996)) containing T7 promoter and terminator. 38^{th} codon encoding asparagine was mutated into amber stop codon by PCR based site-directed mutagenesis. Figure 3 shows the expression vector pK7 which codes human EPO cDNA.

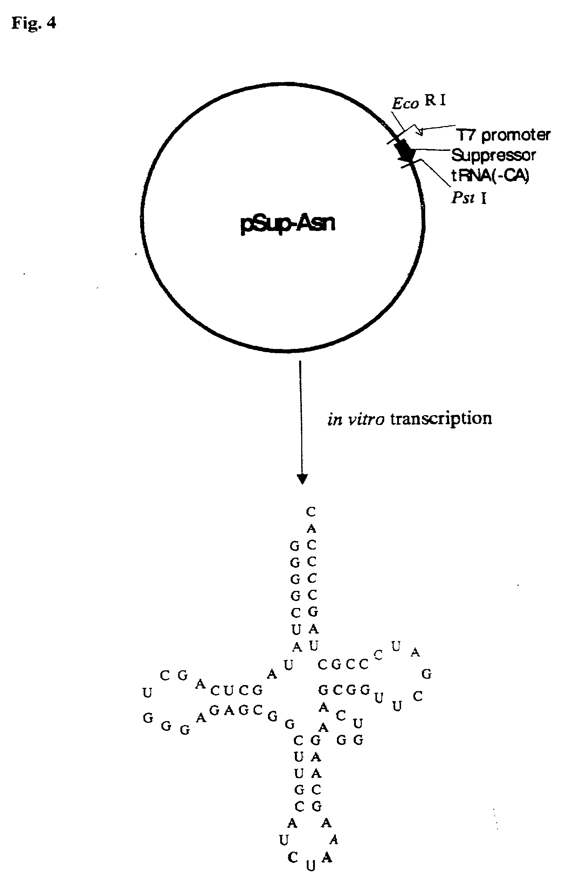

Suppressor tRNA was produced in two steps. Basic structure of the suppressor tRNA was adopted from alanyl tRNA of *E. coli* and the anticodon part of the alanyl tRNA was modified to be cognate to amber stop codon. Other than alanyl tRNA of *E. coli* can also be used to suppress the termination of the translation at the amber or other stop codon. Synthetic DNA coding amber suppressor without terminal CA (tRNA(-CA)) was cloned into pUC19 (pSup-ala). And *Fok* I digested said plasmid was run-off transcribed to produce tRNA(-CA). Figure 4 shows the schematic process of synthesizing tRNA(-CA) from pSup-ala plasmid.

Transcription was performed using RiboMAX large scale RNA production system (Promega) as manufacturer's description. Terminal dinucleotide pdCpA part was synthesized (Robertson, SA et al., Nucleic Acids Res. 17:9649-9660(1989)) and cyanomethyl ester of α-amine and functional group protected amino acid was synthesized.

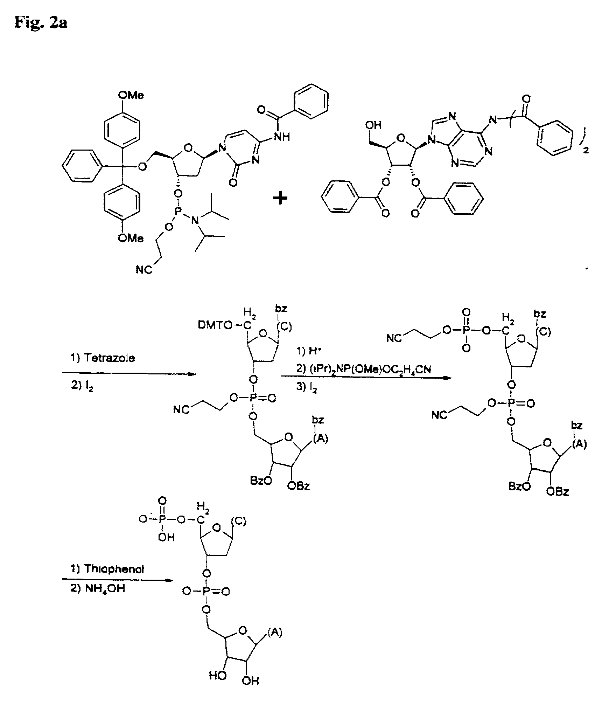

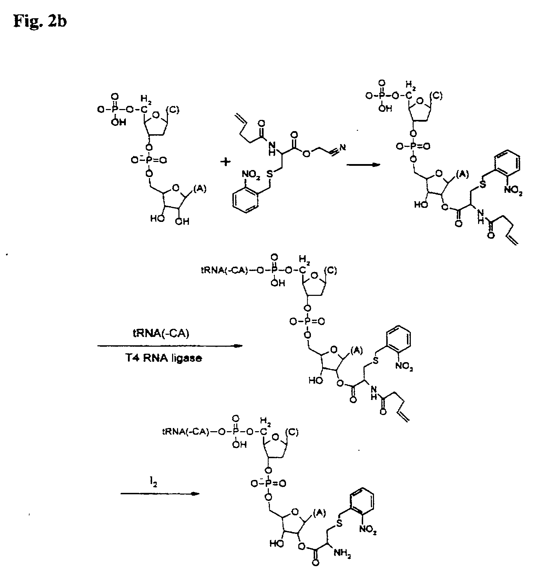

Unnatural amino acid cyanomethyl ester used in this example was N-(4-pentenoyl), S-(2-nitrobenzyl) cysteine cyanomethyl ester. Briefly, pdCpA was synthesized using phosphoramidite chemistry and said unnatural amino acid was synthesized from the reaction of cysteine with 2-nitrobenzyl chloride followed by the reaction with 4-pentenoic anhydride. Active ester of said unnatural amino acid was synthesized by the reaction of said amino acid with chloroacetonitrile. Tetrabutylammonium salt of pdCpA was aminoacylated with active ester of N-(4-petenoyl), S-(2-nitrobenzyl) and purified by HPLC using C₁₈ column. Resulting pdCpA-amino acid complex was ligated to tRNA(-CA) using T4 RNA ligase then 4-pentenoyl protecting group was detached by iodine to a complete suppressor. Figures 2a and 2b show the processes of preparing suppressor tRNA by bonding pdCpA-amino acid complex to tRNA(-CA).

Cell-free protein synthesis and suppression reaction was carried out as basically described in (Kim, D.M. et al., Eur. J. Biochem. 233:881-88b (1996)) with some modifications. The reaction mixture contained 57mM Hepes/KOH pH 8.2, 1.2mM ATP. 0.85mM each of GTP, UTP and CTP, 150mM potassium glutamate, 80mM ammonium acetate, 18mM magnesium acetate, 0.17 mg/ml *E. coli* total tRNA mixture (from strain MRE 600), 34 mg/ml 1-5-formyl-5,6,7,8-tetrahydrofolic acid, 6.7 µg/ml circular plasmid, 33 µg/ml T7 RNA polymerase, 0.3 U/ml pyruvate kinase, 28mM of phospho(*enol*)pyruvate, 0.1 µg/ml suppressor and 20% (v/v) S30 extract. Reaction mixture was incubated at 37°C for 60 minutes. S-(2-nitrobenzyl)cysteinyl-erythropoietin was immuno-purified with monoclonal antibody to EPO using general procedure.

After dialysis in refolding solution (50mM sodium dihydrogenphosphate, 2%(v/v) sodium lauryl sarcosylate, 40µM cupric sulfate) EPO was lyophilized. Samples (1 ml, 50 µg protein in water or PBS) of immuno-purified mutein of EPO in Pyrex test tubes that had been evacuated and closed were irradiated with 320-nm UV. Photodeprotection yielded free sulfhydryl group in the mutein of EPO and this residue (38^{th} cysteine) was used to PEGylate mutein of EPO site-specifically. Photodeprotection and PEGylation were performed in separate manner or simultaneously. Reaction of the muteins of EPO with PEG-maleimide was carried out in 50mM sodium acetate buffer, pH 6.0, containing 5mM EDTA, at a mutein of EPO/PEG molar ratio *of 1:5,* and stirred for 24 hours at room temperature. PEGylated mutein of EPO was purified from unmodified mutein of EPO and PEG by its size using size exclusion chromatography. Purified PEGylated mutein of EPO was subjected to activity test described as follow.

The biological activities of the modified mutein of EPO *in vitro* and *in vivo* were assayed by the growth of the EPO-dependent human cell-line, TF-1, cultured in RPMI 1640 medium containing 10% fetal calf serum (Kitamura, T. et al., Blood 73:375-380 (1989)) and by the incorporation of ⁵⁹Fe into erythroblast cells of exhypoxic polycythemic mice (Goldwasser, E. and Gross, M., Methods in Enzymol. 37:109-121 (1975)), respectively. Values were determined by a parallel line assay (Dunn, C.D.R. and Napier, J.A.P., Exp. Hematol. (N.Y.) 6:577-584 (1978)) using nine doses/sample and two wells/dose for the *in vitro* assay, and more than three doses/sample and three mice/dose for the *in vivo* assay. Any additives in the modified mutein of EPO preparations, such as salts, did not interfere with the assay when used at 1/500 dilution with the medium. The highly purified recombinant human erythropoietin (rHuEPO) calibrated by the second International Reference Preparation was used as standard. As a result, the modified mutein of EPO showed two times higher *in vitro* activity than the intact rHuEPO. And the unmodified mutein of EPO showed three times higher *in vitro* activity than the intact rHuEPO. The *in vivo* biological activity of unmodified mutein of EPO disappeared. The clearance system might work against the unmodified mutein of EPO. And the modified mutein of EPO showed an enhanced *in vitro* activity but similar. to or somewhat low *in vivo* activity compared with the intact rHuEPO. Small fluctuations in *in vivo* activity of the modified mutein of EPO may be due to the extent of hydration of PEG molecule.

### Example 2: Production of PEGylated mutein of erythropoietin by genetic approach

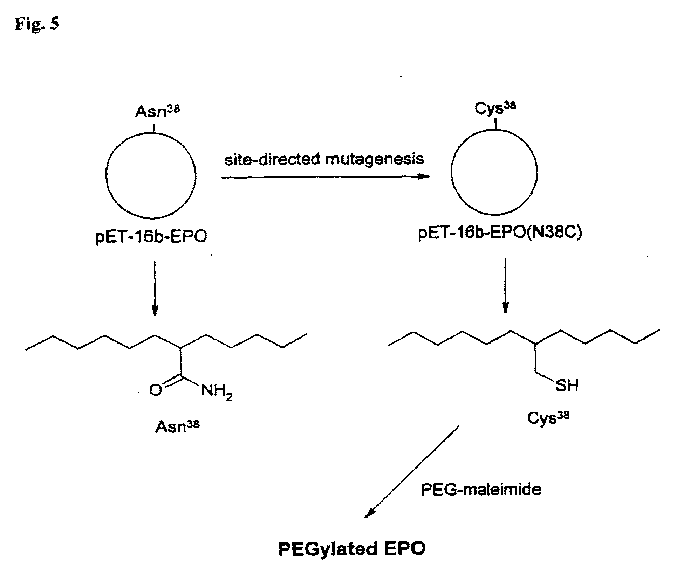

Figure 5 shows the process of preparing PEGlayted mutein of EPO by introducing cysteine at the site of 38^{th} using sited-directed mtagenesis and adding PEG thereto.

Site-directed mutagenesis was carried out using QuickChange™ site-directed mutagenesis Kit acquired from STRATAGENE. A 32-mer oligonucleotide primer of the sequence was chemically synthesized as shown below.

5'-GACGCTTGAATGAGTGTATCACTGTCCCAGAC-3'

This primer was designed to replace asparagine at position 38 of the native human EPO cDNA sequence with cysteine. Plasmid pET16b-hEPO was used as a template. Using the above primer and template, PCR was carried out with following PCR condition: the 50 µℓ reaction mixture contained 5 µℓ of 10 × PCR buffer (Promega), 1 µℓ of 10mM deoxynucleoside triphosphate mixture, 50 pmol of each oligonucleotide, 2.5 units of *Pfu* DNA polymerase, and 50 ng of template. And then 30 µℓ of mineral oil was overlaid. After the completion of PCR, the reaction mixture was cooled to a temperature below 37°C by leaving it in ice for 2 minutes. To digest the template, 1µl of *Dpn* I restriction enzyme was introduced and the mixture was incubated at 37°C for 1 hour.

DNA treated with 1µl *Dpn* I restriction enzyme is transformed into *Epicurian Coli* XL-1 Blue supercompetent cell. The cells were spread onto an ALB plate and the transformant was inoculated into a liquid culture for plasmid preparation.

The N38C hEPO mutein was expressed in *E. coli* BL21(DE3). Cells were grown in NZCYM medium at 37°C until the absorbance at 600-nm reached 0.6 and then 1mM isopropylthio-β-D-galactoside (IPTG) was added to induce expression. 4 Hours after IPTG induction, the cells were centrifuged at 5,000×g for 5 minutes at 4°C. The pellet was suspended in 1 × Binding buffer (without denaturant: 5mM imidazole, 0.5 M NaCl, 50mM sodium phosphate, pH 8.0). The cells were lysed by sonication. The resultant mixture was centrifuged at 7,000×g for 40 minutes at 4°C. The pellet was suspended in 1 × Binding buffer and then sonicated. The mixture was centrifuged at the same condition. The pellet was re-suspended in 1 × Binding buffer (with denaturant: 5mM imidazole, 0.5 M NaCl, 50mM sodium phosphate, 8 M urea, pH 8.0). The suspension was incubated on ice for 1 hour and then centrifuged at 18,000×g for 40 minutes at 4°C. The supernatant was filtered with 0.45µm syringe filter.

For purification of the expressed hEPO, Ni-NTA affinity chromatography was carried out by the addition of buffers in the following order:
1. 1 × Binding buffer (with denaturant) for equilibration,
2. Cell extract,
3. 1 × Binding buffer (with denaturant),
4. 1 × Wash buffer (20mM imidazole, 0.5 M NaCl, 50mM sodium phosphate, 8 M urea, pH 8.0),
5. Elution buffer (1 M imidazole, 0.5 M NaCl, 50mM sodium phosphate, 8 M urea, pH 8.0)

From purified mutein of EPO, poly(histidine) tag was removed by the action of factor Xa. Reaction mixture contained 20mM Tris-HCl (pH 7.4), 0.1M NaCl and further purified by HPLC on C₁₈ column.

3mM dithiothreitol was added to the protein solution containing 8M urea, and then the mixture dialyzed in refolding solution (50mM sodium dihydrogenphosphate, 2%(v/v) sodium lauryl sarcosylate, 40µM cupric sulfate). Then the buffer was replaced by a PEGylation buffer (50mM sodium acetate, 5mM EDTA, pH 6.0). Reaction of the muteins of EPO with PEG-maleimide was carried out in 50mM sodium acetate buffer, pH 6.0, containing 5mM EDTA, at a mutein of EPO/PEG molar ratio of 1:5, and stirred for 24 hours at room temperature. PEGylated mutein of EPO was purified from unmodified mutein of EPO and PEG by its size using size exclusion chromatography. Purified PEGylated mutein of EPO was subjected to activity test described as follow.

The biological activities of the modified mutein of EPO *in vitro* and *in vivo* were assayed by the growth of the EPO-dependent human cell-line, TF-1, cultured in RPMI 1640 medium containing 10% fetal calf serum (Kitamura, T. et al., Blood 73:375-380 (1989)) and by the incorporation of ⁵⁹Fe into erythroblast cells of exhypoxic polycythemic mice (Goldwasser, E. and Gross, M., Methods in Enzymol. 37:109-121 (1975)), respectively. Values were determined by a parallel line assay (Dunn, C.D.R. and Napier, J.A.P., Exp. Hematol. (N.Y.) 6:577-584 (1978)) using nine doses/sample and two wells/dose for the *in vitro* assay, and more than three doses/sample and three mice/dose for the *in vivo* assay. Any additives in the modified mutein of EPO preparations, such as salts, did not interfere with the assay when used at 1/500 dilution with the medium. The highly purified recombinant human erythropoietin (rHuEPO) calibrated by the second International Reference Preparation was used as standard. As a result, the modified mutein of EPO showed two times higher *in vitro* activity than the intact rHuEPO. And the unmodified mutein of EPO showed three times higher *in vitro* activity than the intact rHuEPO. The *in vivo* biological activity of unmodified mutein of EPO disappeared. The clearance system might work against the unmodified mutein of EPO. And the modified mutein of EPO showed an enhanced *in vitro* activity but similar to or somewhat low *in vivo* activity compared with the intact rHuEPO. Small fluctuations in *in vivo* activity of the modified mutein of EPO may be due to the extent of hydration of PEG molecule.

### EQUIVALENTS

Those skilled in the art will recognize, or be able to ascertain using no more than routine experimentation, many equivalents to specific embodiments of the invention described specifically herein. Such equivalents are intended to be encompassed in the scope of the following claims.

## Claims

1. A method of producing biologically active modified muteins of erythropoietin (EPO) comprising;
converting the codon corresponding to amino acid residue at the site to be modified to an amber stop codon, other stop codon, or frameshift codon containing rare codon by site-directed mutagenesis,
during cell-free protein synthesis, including a suppressor tRNA corresponding to the amber stop codon, other stop codon, or frameshift codon containing rare codon therein to attach an unnatural amino acid attached to tRNA to EPO protein, and thereby preparing a mutein of EPO (wherein, the below modifier may be already attached to the unnatural amino acid), and
incorporating a modifier(s) to one or more desired sites on the mutein of EPO via chemical, enzymatic, or chemo-enzymatic reaction to modify the mutein.

2. The method according to claim 1, wherein said modifier is PEGs, monosaccharide, disaccharides, oligosaccharides and/or polysaccharides.

3. Selectively modified muteins of EPO prepared by the method according to claim 1 or 2.

4. A method of producing biologically active modified muteins of EPO comprising;
converting a codon corresponding to certain amino acid at the site to be modified to a cysteine coding codon,
transforming host cell with the above mutant DNA to prepare cysteine-introduced muteins of EPO containing free sulfhydryl group at the site to be modified,
bonding a modifier(s) to free sulfhydryl groups on the mutein of EPO via chemical, enzymatic, or chemo-enzymatic reaction to incorporate the modifiers to one or more desired sites of the mutein.

5. The method according to claim 4, wherein said modifier is PEGs, monosaccharide, disaccharides, oligosaccharides and/or polysaccharides.

6. Selectively modified muteins of EPO prepared by the method according to claim 4 or 5.


### Annex to the application documents - subsequently filed sequences listing

SEQUENCE LISTING

<110> DREAMBIOGEN CO., LTD.

<120> Modified muteins of EPO derived from in vitro or in
      vivo expression system of microorganism

<130> 11009EP2

<140> 01420032.3
<141> 2001-02-13

<150> KR0082531
<151> 2000-12-27

<160> 5

<170> PatentIn Ver. 2.1

<210> 1
<211> 501
<212> DNA
<213> Homo sapiens

<220>
<221> mutation
<222> (111)..(113)
<223> introduction of cysteine codon by site-directed
      mutagenesis in the EPO gene

<400> 1
gccccaccac gcctcatctg tgacagccga gtcctggaga ggtacctctt ggaggccaag 60
gaggccgaga atatcacgac gggctgtgct gaacactgca gcttgaatga gtgtatcact 120
gtcccagaca ccaaagttaa tttctatgcc tggaagagga tggaggtcgg gcagcaggcc 180
gtagaagtct ggcagggcct ggccctgctg tcggaagctg tcctgcgggg ccaggccctg 240
ttggtcaact cttcccagcc gtgggagccc ctgcagctgc atgtggataa agccgtcagt 300
ggccttcgca gcctcaccac tctgcttcgg gctctgggag cccagaagga agccatctcc 360
cctccagatg cggcctcagc tgctccactc cgaacaatca ctgctgacac tttccgcaaa 420
ctcttccgag tctactccaa tttcctccgg ggaaagctga agctgtacac aggggaggcc 480
tgcaggacag gggacagatg a                                           501

<210> 2
<211> 501
<212> DNA
<213> Homo sapiens

<220>

<221> mutation
<222> (111)..(113)
<223> introduction of a amber codon by site-directed
      mutagenesis in the EPO gene

<400> 2
gccccaccac gcctcatctg tgacagccga gtcctggaga ggtacctctt ggaggccaag 60
gaggccgaga atatcacgac gggctgtgct gaacactgca gcttgaatga gtagatcact 120
gtcccagaca ccaaagttaa tttctatgcc tggaagagga tggaggtcgg gcagcaggcc 180
gtagaagtct ggcagggcct ggccctgctg tcggaagctg tcctgcgggg ccaggccctg 240
ttggtcaact cttcccagcc gtgggagccc ctgcagctgc atgtggataa agccgtcagt 300
ggccttcgca gcctcaccac tctgcttcgg gctctgggag cccagaagga agccatctcc 360
cctccagatg cggcctcagc tgctccactc cgaacaatca ctgctgacac tttccgcaaa 420
ctcttccgag tctactccaa tttcctccgg ggaaagctga agctgtacac aggggaggcc 480
tgcaggacag gggacagatg a                                           501

<210> 3
<211> 166
<212> PRT
<213> Homo sapiens

<220>

<400> 3
Ala Pro Pro Arg Leu Ile Cys Asp Ser Arg Val Leu Glu Arg Tyr Leu
1               5                   10                  15

Leu Glu Ala Lys Glu Ala Glu Asn Ile Thr Thr Gly Cys Ala Glu His
            20                  25                  30

Cys Ser Leu Asn Glu Cys Ile Thr Val Pro Asp Thr Lys Val Asn Phe
        35                  40                  45

Tyr Ala Trp Lys Arg Met Glu Val Gly Gln Gln Ala Val Glu Val Trp
    50                  55                  60

Gln Gly Leu Ala Leu Leu Ser Glu Ala Val Leu Arg Gly Gln Ala Leu
65                  70                  75                  80

Leu Val Asn Ser Ser Gln Pro Trp Glu Pro Leu Gln Leu His Val Asp
                85                  90                  95

Lys Ala Val Ser Gly Leu Arg Ser Leu Thr Thr Leu Leu Arg Ala Leu
            100                 105                 110

Gly Ala Gln Lys Glu Ala Ile Ser Pro Pro Asp Ala Ala Ser Ala Ala
        115                 120                 125

Pro Leu Arg Thr Ile Thr Ala Asp Thr Phe Arg Lys Leu Phe Arg Val
    130                 135                 140

Tyr Ser Asn Phe Leu Arg Gly Lys Leu Lys Leu Tyr Thr Gly Glu Ala
145                 150                 155                 160

Cys Arg Thr Gly Asp Arg
                165

<210> 4
<211> 73
<212> RNA
<213> Escherichia coli

<220>
<221> mutation
<222> (33)..(35)
<223> alanyl tRNA with the anticodon part modified to be
      cognate to amber stop codon

<220>
<221> modified_base
<222> (72)
<223> 72-base is deoxy-cytidine form

<400> 4
ggggcuauag cucagcuggg agagcgcuug caucuaaagc aagaggucag cgguucgauc 60
ccgcuuagcc cca                                                    73

<210> 5
<211> 32
<212> DNA
<213> Artificial Sequence

<220>
<223> Description of Artificial Sequence: chemically
      synthesized primer used in site-directed
      mutagenesisto replace asparagin innative human
      EPOcDNA  sequence with cystein

<400> 5
gacgcttgaa tgagtgtatc actgtcccag ac                               32